# EUROPEAN PATENT SPECIFICATION

(11) **EP 1 472 339 B1**
(45) Date of publication and mention of the grant of the patent: **19.03.2008**
(21) Application number: 02711197.0
(22) Date of filing: 08.02.2002
(51) Int. Cl.: C12N 1/21, C12N 15/00, C12Q 1/00, C12Q 1/68

(54) **A PROCESS FOR IDENTIFYING A NOVEL TARGET FOR USE FOR THE DEVELOPMENT OF THERAPEUTIC MODALITIES AND DRUGS EFFECTIVE AGAINST TUBERCULOSIS**
VERFAHREN ZUR IDENTIFIZIERUNG EINES NEUEN ZIELS ZUR VERWENDUNG BEI DER ENTWICKLUNG VON GEGEN TUBERKULOSE WIRKSAMEN THERAPEUTISCHEN MODALITÄTEN UND ARZNEISTOFFEN
PROCEDE D'IDENTIFICATION D'UNE NOUVELLE CIBLE UTILISEE POUR LE DEVELOPPEMENT DE MODALITES THERAPEUTIQUES ET DE MEDICAMENTS PERMETTANT DE LUTTER EFFICACEMENT CONTRE LA TUBERCULOSE

(43) Date of publication of application: 03.11.2004
(73) Proprietor: The Director, all India Institute of Medical Science, New Delhi 110 029, Maharashtra (IN); The Secretary, Department of Biotechnology, New Delhi 110 003, Maharashtra (IN)
(72) Inventor: TYAGI, Jaya Sivaswami Dpt. of Biotechnology, New Delhi 110 029 Maharashtra (IN); KAPUR, Vandana Dpt. of Biotechnology, New Delhi 110 029 Maharashtra (IN)
(74) Representative: Kaiser, Magnus
(86) International application number: PCT/IN2002/000022
(87) International publication number: WO 2003/066838

(56) References cited:
- DATABASE MEDLINE [Online] DASGUPTA N. ET AL: 'Identification of a restriction fragment length polymorphism associated with a deletion that maps in a transcriptionally active open-reading frame, orfX, in mycobacterium tuberculosis Erdman', XP002978871 Database accession no. (NLM10645444) & TUBERCLE AND LUNG DISEASE vol. 79, no. 2, 1998, pages 75 - 81
- DATABASE MEDLINE [Online] DASGUPTA N. ET AL: 'Characterization of a two-component system, devR-devS, of mycobacterium tuberculosis', XP002978872 Database accession no. (NLM10970762) & TUBERCLE AND LUNG DISEASE vol. 80, no. 3, 2000, pages 141 - 159
- PEREZ E. ET AL: 'An essential role for phoP in mycobacterium tuberculosis virulence' MOLECULAR BIOLOGY vol. 41, no. 1, 2000, pages 179 - 187, XP002978890

## Description

This invention relates to a process for identifying a novel target for use for the development of therapeutic modalities and drugs effective against tuberculosis. Specifically, this invention relates to a process for identifying new and novel target for the development of therapeutic modalities including anti tubercular drugs with special reference to mycobacterial existence persistence in hypoxia.

Tuberculosis is the leading cause of death from a single infectious agent killing more than 3 million people per year worldwide. In the year 1998, the estimated number of TB cases in India was 2,078,076 among which 935,134 cases were likely to be infectious. The consequence of infection is clearly an outcome of the continuous interplay between the pathogen and the host immune defence. In most instances, the infected individual mounts an effective immune response that culminates in granuloma formation around the infective foci and cessation of disease progression. The environment within granulomas is predicted to be hypoxia. Clinical studies suggest that the bacilli within these granulomas are not killed but instead remain dormant. This is termed a latent infection. Approximately 10% of latent infections reactivate, resulting in active infectious disease months to years after initial infection. The large number of latently infected individuals presents a major impediment to reducing the incidence of tuberculosis and the rate of *M.Tuberculosis* transmission. The adaptation of *M.tuberculosis* during the spectrum of infection and disease is likely implemented through precise genetic pathways that are modulated by specific physiological and environmental conditions within host tissues.

There is an urgent need to understand these pathways in order to devise novel and more directed strategies for the prevention, control and treatment of tuberculosis. Conventional drugs target pathways required for bacterial growth and division such as cell-wall biosynthesis and DNA replication. Their poor activity against slow-growing or non-growing bacteria is thought to be an important reason why currently used regimens take so long to eradicate infection.

The *devR-devS* genes, designated as *Rv3133c* and *Rv3132c* respectively in the annotated *M.tuberculosis* genome are predicted to encode a response regulator, DevR, and a histidine kinase sensor, DevS, respectively. This genetic system was identified earlier in our laboratory by subtractive hybridization using RNA from virulent and avirulent strains of *M.tuberculosis* (Ref.1). Here we describe the process of identifying this system as a new and novel target for the development of therapeutic modalities including anti tubercular drugs with special reference to mycobacterial persistence existence in hypoxia.

Therefore the main object of the invention is to identify the target which is responsible for the recurrence/reactivation of the disease in the patient or that which enables the organism to adapt to hypoxia..

Another object of this invention is to identify the target responsible for the recurrence/reactivation of the disease or that which enables the organism to adapt to hypoxia and to develop the therapeutic modalities and anti tubercular drugs.

According to this invention there is provided a process for identifying a novel target for use for the development of therapeutic modalities and drugs effective against tuberculosis comprising: -
I. disrupting *devR* gene located in a ∼3.3 kb EcoRI-HindIII insert of plasmid pJT53.34 with kanamycin resistance (KmR) cassette,
II. constructing pJQ200SkdevR::kan from the disrupted devR gene,
III. introducing said plasmid into *M. tuberculosis* H37Rv by electroporation,
IV. selecting single crossover transformants indicative of plasmid integration on middle brook 7H10 agar plates containing 20 µg/ml kanamycin,
V. analyzing the same by polymerase chain reaction (PCR) for the presence of devR, Km^{R} and sucrose resistance *SacB* gene sequences,
VI. subjecting said sequences to the step of Southern analysis with *devR* probe, *devS* probe kanamycin resistant gene probe so as to designate *M. tuberculosis* Dup devR containing wild-type and the disrupted copies of the devR locus,
VII. growing *M.tuberculosis* Dup *devR* in middle brook 7H9 medium containing kanamycin 20 µg/ml and 2% sucrose for 7 days,
VIII. subjecting said grown *M.tuberculosis* Dup *devR* strain into a plurality of plates having a medium middle brook 7H10 medium containing kanamycin 20 µg/ml and 2% sucrose therein so as to obtain kanamycin resistant transformants,
IX. subjecting said grown *M.tuberculosis* devR to the step of Southern hybridisation followed by polymerase chain reaction process for the confirmation of said allelic exchange,
X. subjecting said transformants to the step of polymerase chain reaction analysis for *devR::*kan disrupted gene,
XI. subjecting said devR kan disrupted gene to the step of Western blotting and immuno electron microscopy for the confirmation of functional disruption of said gene,
XII. evaluating the viability of growth of the strain *M tuberculosis devR* mutant under conditions of oxygen limitation for *devR* and *devS* gene expression,
XIII. evaluating the growth and viability of said strain *M.tuberculosis devR* mutant under conditions of oxygen limitation in aerobic conditions for devR and devS gene expression,
XIV. subjecting said grown strain to the step of RT-PCR analysis for transcripts obtained from the Rv3134c-devR-devS operon,
XV. scanning said transcripts by using the Ultra-Violet products gel documentation system and subjecting the same to the step of densitometric analysis by using a computer software,
XVI. testing *M.tuberculosis* devR mutant strain for virulence in guinea pigs.

The process for identifying a novel target for use for the development of therapeutic modalities and drugs effective against tuberculosis is herein described in detail with the help of the accompanying drawings wherein:-
Fig.1 shows the construction of devR mutant strain of *M.tuberculosis*
   a) Southern hybridization analysis of recombinant *M.tuberculosis* strains.
   b) PCR analysis of a representative devR mutant clone.
Fig.2 shows
   a) Western blot analysis of devR mutant *M. tuberculosis*
   b) Immuno electron microscopy of devR mutant *M.tuberculosis*
   c) In vitro morphological analysis of the devR mutant and H37Rv strains of *M tuberculosis.*
Fig. 3 shows expression analysis ofRv3134c-devR-devS operon in wild-type and mutant strains of *M.tuberculosis.*
Fig.4 shows characteristics of liver and lung granuloma in guinea pigs infected with devR mutant and H37Rv strains of *M.tuberculosis*

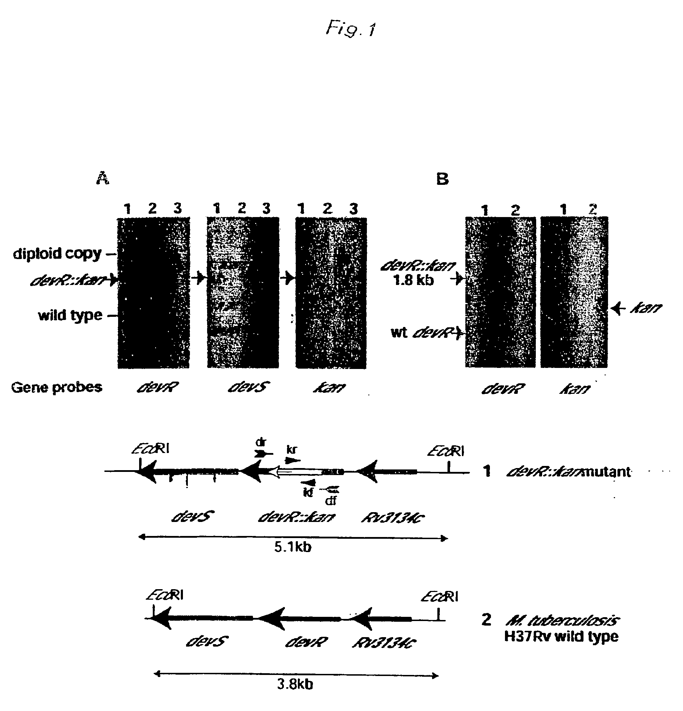

As per the process of this invention the devR gene of *M.tuberculosis* is disrupted by allelic exchange using standard technologies. Briefly, the devR gene located in a ∼3.3 kb *Eco*RI*-Hind*III insert of plasmid pJT53.34 is disrupted with a kanamycin resistance (Km^{R}) gene at a unique *Ppu*MI site. The disrupted devR allele is excised as an *Apa*I-*Bam*HI fragment and cloned into the corresponding sites of plasmid pJQ200SK constructing pJQ200Sk*devR::kan.* This plasmid is introduced into *M.tuberculosis* H37Rv by electroporation. Single crossover transformants indicative of plasmid integration are selected on middle brook 7H10 agar plates containing 20µg/ml kanamycin and analyzed by polymerase chain reaction (PCR) for the presence of *devR,* Km^{R} and sucrose (sacB) gene sequences. Twenty five Km^{R} colonies are analysed and only 6 are positive by PCR for Km^{R}, signifying a high frequency of spontaneous Km^{R} (∼75%). All 6 colones are positive for sacB PCR indicating the involvement of a single crossover event in the generation of Km^{R} colonies. Three (of 6) are positive by PCR for the wild-type *devR* (513-bp) and the *devR::kan* products (1.8-kb) suggesting that integration of the plasmid-borne *devR::kan* copy into the *M.tuberculosis* chromosome had occurred. PCR assays using chromosome-flanking primers together with Km^{R} gene-specific primers suggested that plasmid integration had occurred near the *devR* or *devS* locus in one clone. Upon Southern hybridisation with the *devR* probe, two signals of 3.8 kb and ∼9.5 kb are obtained in contrast to a single hybridization signal of 3.8 kb obtained in the parental strain. Identical results are obtained using the *devS* gene as probe (Fig.1A). Hybridization with the Km^{R} gene probe highlighted the ∼9.5 kb fragment containing the vector backbone resulting from a single crossover recombination event to the left of the Km^{R} gene in the *devR or devS* locus. The merodiploid strain containing the wild-type and the disrupted copies of the devR locus is designated as *M.tuberculosis* Dup *devR* (Fig.1A).

The allelic exchange event is selected in the second step by growing *M.tuberculosis* Dup *devR* in middle brook 7H9 medium containing kanamycin (20 µg/ml) and 2% sucrose for 7 days. Serial dilutions are plated on 7H10 medium containing kanamycin (20 µg/ml) and 2% sucrose. A total of 87 Km^{R} and sucrose resistant (Suc^{R}) transformants are obtained; 31 of these are negative by PCR for wild-type *devR* gene, 18 of which are positive by PCR for the *devR::kan* disrupted gene. Sucrose resistance arose from one of two events; the sacB gene is either lost as a result of the resolution (64%) or have accumulated mutation(s) resulting in the loss of function (36%). Allelic exchange is confirmed by Southern hybridization. Briefly, 16 (out of 18) Km^{R} - Suc^{R} transformants are probed with *devR* and *devS* gene probes. A double crossover event occurred in 15 clones leading to the presence of *devR::kan* copy (5.1 kb hyridization signal) instead of the wild-type *devR* gene (3.8 kb signal), the mutants having a size increment of 1.3 kb corresponding to the Km^{R} cassette inserted in the *devR* locus. Hybridization with the Km^{R} gene probe confirmed the retention of Km^{R} cassette-disrupted *devR* gene copy on the chromosome. The lack of hybridization with *sacB* probe established that the gene has lost during resolution of the tandem duplication (not shown). A representative clone is shown in Fig.1A. One *devR* knockout clone is randomly selected for further characterization. The gene replacement is also confirmed by PCR (Fig.1B).

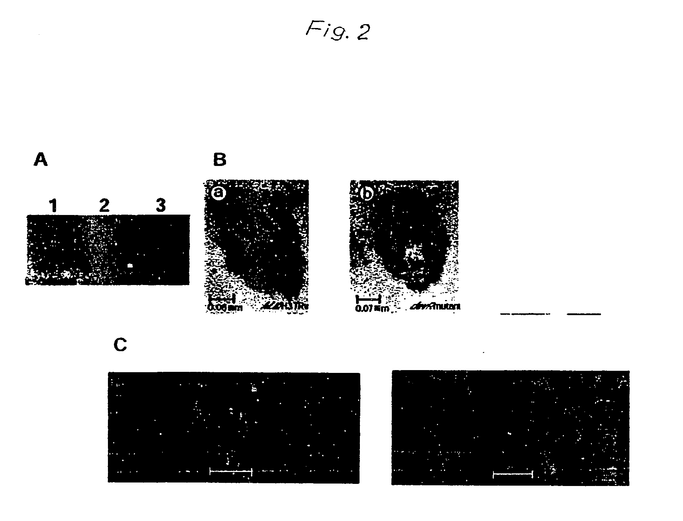

Functional disruption of the *devR* gene in the mutant is confirmed by Western blotting and immuno electron microscopy using standard procedures. Sonicates of logarithmic phase cultures of *M.tuberculosis* H37Rv, *devR* mutant and *E*. *coli* are subjected to denaturing polyacrylamide gel electrophoresis, proteins transferred to nitrocellulose membrane and probed with polyclonal anti-DevR antibody diluted 1:1,000 raised in rabbits against DevR protein of *M.tuber*c*ulosis*. Immunoreactivity is assessed using horseradish peroxidase-conjugated donkey anti-rabbit immunoglobulin G and 3,3'-diamino-benzidine substrate. DevR protein is visualized in *M.tuherculosis* H37Rv and in recombinant *E.coli* overexpressing DevR protein but not in the mutant strain (Fig.2A). By immuno electron microscopy, DevR labelling occurred on the surface and in the cytosol of wild-type organisms (25 ± 2.4 gold grains per bacillus) but not in the mutant strain (2 ± 0.88 gold grains per bacillus, Fig.2B).

The morphology of mutant and parental bacilli is compared by scanning electron microscopy. Logarithmic phase mutant bacilli are longer in size (average 4-6 µm) in comparison to the wild-type bacilli (average 2-4 µm, Fig.2C). The growth curves of the *devR* mutant and parental strains cultured in triplicate under aerobic conditions in 7H9 containing 10% albumin-dextrose and 0.05%. Tween 80 are compared. The mean A₅₉₀ increased from 0.054 on day 1 to 1.41 on day 28 with wild-type cultures and from 0.048 on day 1 to 1.9 on day 28 for mutant cultures. The differences in A₅₉₀ at various time points for the mutant and parental strains are not statistically significant (by Wilcoxon Rank Sum test) suggesting that the *devR* mutation did not have a significant impact on the growth of *M.tuberculosis* under aerobic conditions.

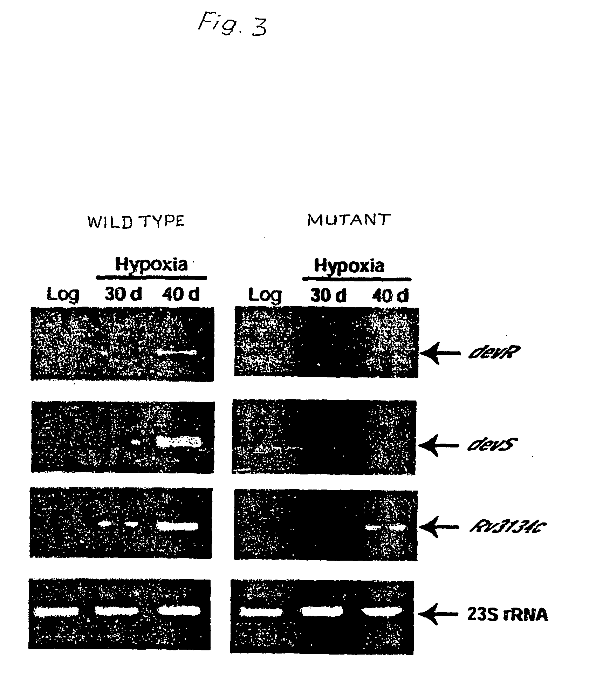

The effect of oxygen limitation on the expression of *devR* and *devS* genes of *M.tuberculosis,* shown earlier to be co transcribed in *M.tuberculosis* was assessed. The use of an *in vitro* model of *M.tuberculosis* dormancy in which the cultures have grown in Dubos Tween Albumin without agitation and slowly descended to the bottom of the culture vessel where the low oxygen concentration limited growth. After -30 days, the bacteria entered a stationary/nonreplicating growth phase. During this period, the wild-type bacteria grew ~18-fold. The wild-type organisms adapted to gradual oxygen deprivation without a detectable loss of viability. The mutant bacteria grew only ~5-fold over the 30 day period. At the end of the experiment, the viability of the mutant strain is ∼25% of that of the parental strain. Aerobic shaker cultures of *M.tuberculosis* parental and mutant strains are grown simultaneously to logarithmic phase (A₅₉₀ ∼ 0.4). RNA is isolated from logarithmic phase, 30-day and 40-day unagitated cultures using the Rneasy Mini kit (Qiagen, Germany) and RT-PCR analysis is performed for transcripts originating from the *Rv3134c-devR-devS* operon (*Rv3134c* is the first gene in this operon). The gels are scanned using the UVP gel documentation system and densitometric analysis is performed using the Labworks^{™} analysis software (Ultra-Violet product, USA). The expression of *devR, devS* and *Rv3134c* genes is up regulated ∼3- to 4-fold in wild-type cultures under hypoxic conditions. An up regulation is also observed in the mutant strain except that the basal level of expression of *Rv3134c* and *devS* gene is ~2.5-fold lower than that observed in the wild-type strain. As expected, transcripts from the wild-type *devR* gene are not detected in the mutant strain (Fig.3). The expression and up regulation of the *devS* gene in the mutant strain is thought to be due to transcription originating upstream since the expression of the Km^{R} cassette (within the *devR* gene) is also up regulated under similar conditions (data not shown).

The process for identifying a novel target for use for the development of therapeutic modalities and anti tubercular drugs is herein described in detail.

The effect of the devR mutation on *in vivo* growth and the ability to cause disease in guinea pigs is evaluated as described. Albino, random bred guinea pigs (five animals per group) are subcutaneously injected with 0.1 ml of viable bacilli in phasphate-buffered saline (*M.tuberculosis* H37Rv x 10⁶ CFU and devR mutant 3.2 x 10⁷ CFU). Guinea pigs were sacrificed 47 days post-infection. One animal (H37Rv group) that died a non-tuberculosis death before the date of sacrifice is omitted from the analysis. The amount of visible tuberculosis in internal organs is scored immediately after sacrifice as described. A heavy involvement of the lungs, liver, spleen and lymph node is noted in the guinea pigs infected with *M.tuberculosis* H37Rv. The visual scores ranged between 43 and 93 (mean 77) and between 23 and 48 (mean 38.4) for guinea pigs infected with the parental and mutant strains respectively, the difference being significant (p<0.05, Table 1). The liver is the most affected organ and heavy invasion with numerous large tubercles and areas of necrosis is seen in guinea pigs infected with the parental strain. Spleen and lungs showed moderate invasion with numerous small tubercles. Considerably less number of visible lesions is seen in the organs of guinea pigs infected with the mutant strain (Table 1). Spleens are homogenized and serial dilutions are plated on LJ slants. A total of 7.09±0.83 log₁₀ cfu are isolated from spleens of animals infected with the parental strain vs. 4.4±1.21 log₁₀ cfu recovered from spleens of animals infected with the mutant strain, the difference being significant (p<0.05, Table 1).

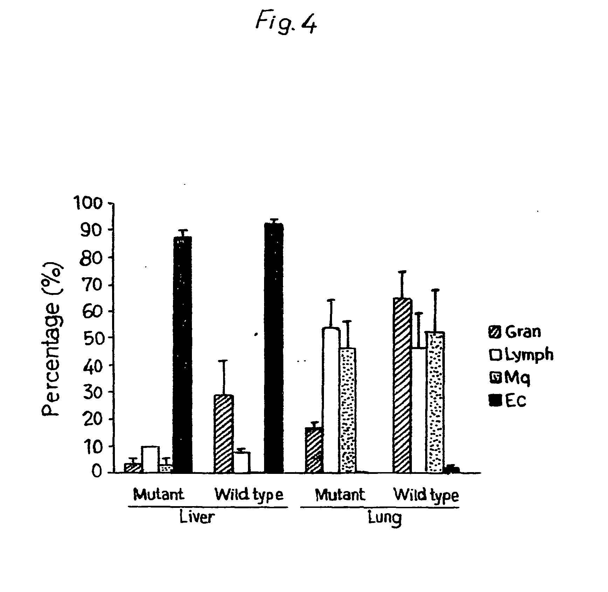

Serial 5 µm sections from the liver and lung autopsy specimens are subjected to a semiquantitative appraisal of the histological features (organ architecture, the percentage area occupied by the granuloma in the section and the percentage of the major cellular components within the granuloma) as described previously. Liver sections from three of five guinea pigs infected with the mutant strain had normal architecture and do not show any granuloma or the presence of inflammatory cell infiltrates. In the remaining two animals infected with the mutant strain, minimal, well-organized, non-necrotic epithelioid cell granuloma is observed. Liver sections from all four guinea pigs infected with the parental strain showed the presence of well-formed granuloma that consisted of epithelioid cells and lymphocytes. Other cell types are absent. In one animal, the granuloma is extensive (65%) and is accompanied by the partial destruction of organ architecture (fig.4). Compared to the liver, much more extensive involvement of the lung is noted. The lung architecture in all the guinea pigs infected with the mutant strain is normal. Although all the animals showed the presence of granuloma, it is minimal and consisted of both lymphocytes and macrophages. Giant cells and other cells or necrosis are not seen in any of the lungs. In animals infected with the parental strain, lung from one guinea pig is completely destroyed and is partially damaged in the remaining three. The granuloma ranged from 40% to 85% and varied from being predominantly lymphocytic to mainly histiocytic (fig.4).

At 7 weeks post-infection, significantly less organ pathology is observed and a nearly thousand-fold lower bacterial load are recovered from guinea pigs infected with the mutant strain compared to those infected with the parental strain. The preponderance of epithelioid cells over macrophages and lymphocytes in liver as compared to lung is suggestive of a good immune response and more advanced resolution of granuloma in the former.

Therefore it is seen that the DevR-DevS two-component system is involved in the virulence of *M.tuberculosis* and could well be a key regulatory link between oxygen limitation and the initiation and maintenance of the adaptive response to hypoxia. Mycobacterial adaptation to an anaerobic microenvironment is thought to provide a means for the tubercle bacilli to reside indefinitely in a dormant/stationery phase-like persistent state within inflammatory and necrotic lesions such as granuloma. Therefore this genetic system could serve as a vital target for the development of new and novel drugs for the treatment of tuberculosis particularly the condition of persistence.

## Claims

1. A process for identifying a novel target for use for the development of therapeutic modalities and drugs effective against tuberculosis comprising the following steps:
I. disrupting devR gene (Rv3133c) located in a ∼ 3.3 kb *EcoRI-HindIII* insert of plasmid pJT53.34,
II. constructing pJQ200Sk*devR::kan* from the disrupted devR gene,
III. introducing said plasmid into *M.tuberculosis* H37Rv,
IV. selecting single crossover transformants indicative of plasmid integration on middle brook 7H10 agar plates containing kanamycin,
V. analyzing the same by polymerase chain reaction (PCR) for the presence of *devR,* Km^{R} and sucrose resistance (SacB) gene sequences,
VI. subjecting said sequences to the step of southern analysis with each of *devR* probe, *devS* probe and Km^{R} gene probe so as to designate *M.tuberculosis* Dup *devR* containing wild-type and the disrupted copies of the devR locus,
VII. growing *M.tuberculosis* Dup *devR* in middle brook 7H9 medium containing kanamycin and sucrose,
VIII. subjecting said grown *M.tuberculosis* Dup *devR* strain into a plurality of plates having a 7H10 medium containing kanamycin and sucrose therein so as to obtain kanamycin resistant transformants, thereby obtaining *devR* mutant strain of *M.tuberculosis,*
IX. subjecting said grown *M.tuberculosis devR* mutant to the step of Southern hybridisation followed by polymerase chain reaction process for the confirmation of replacement of wild-type *devR* allelic with disrupted *devR* allelic,
X. subjecting said *M.tuberculosis devR* transformants to the step of polymerase chain reaction analysis for devR kan disrupted gene,
XI. subjecting said devR kan disrupted gene to the step of Western blotting and immuno electron microscopy for the confirmation of functional disruption of said gene,
XII. evaluating the viability of growth of the strain *M.tuberculosis devR* mutant under conditions of oxygen limitation for *devR* and *devS* gene expression,
XIII. evaluating the viability of growth of said strain *M.tuberculosis devR* mutant under conditions of oxygen limitation in aerobic conditions for *devR* and *devS* gene expression.
XIV. subjecting said grown strain to the step of RT-PCR analysis for transcripts obtained from the *Rv3134c-devR-devS* operon,
XV. scanning said transcripts by using an ultraviolet transilluminator and gel documentation system and subjecting the same to the step of densitometric analysis by using a computer software,
XVI. testing *M.tuberculosis devR* mutant strain for virulence in non-human animals.

2. The process according to claim 1, wherein the testing for virulence is performed in guinea pigs and comprises:
(a) histopathological analysis of the infected organs from guinea pigs infected with *devR* mutant and wild-type strains *of M.tuberculosis,* and
(b) recovery of *M.tuberculosis* from spleens of infected animals and quantification of bacterial load.

3. The process according to claim 1 or 2, wherein the said *devR* allele is disrupted with a kanamycin resistance (Km^{R}) gene at a unique *Ppu*MI site.

4. The process according to any of claims 1 to 3, wherein the *devR* allele is excised as an *Apa*I*-Bam*HI fragment and cloned into the corresponding sites of plasmid pJQ200SK so as to construct pJQ200SK*devR::kan.*

5. The process according to any of claims 1 to 4, wherein the plasmid is introduced into *M.tuberculosis* H37Rv by electroporation.

6. A *devR* mutant strain of *M.tuberculosis,* wherein the wild-type *devR* gene is disrupted with an antibiotic resistance gene.

7. A *devR* mutant strain of *M.tuberculosis,* obtained by the process as defined in any of claims 1 to 5.

8. A *devR* mutant strain of *M. tuberculosis,* obtainable by a process comprising the following steps:
I. disrupting the wild-type *devR* gene (*Rv3133c*) located in a ∼ 3.3 kb *Eco*RI-*Hin*dIII insert of plasmid pJT53.34 with an antibiotic resistance gene at unique *Ppu*MI site,
II. excising the disrupted *devR* allele as an *Apa*I-*Bam*HI fragment and cloning the same into the corresponding sites of plasmid pJQ200SK,
III. introducing the plasmid obtained in step II into wild-type *M. tuberculosis* H37Rv,
IV. selecting single crossover transformants indicative of plasmid integration,
V. cultivating single crossover transformants selected in step IV and selecting double crossover transformants being unable to express the wild-type *devR* gene, thereby obtaining a *devR* mutant strain of *M.tuberculosis.*

9. The *devR* mutant strain according to claim 6 or 8, wherein the antibiotic resistance gene is a kanamycin resistance gene.

10. The *devR* mutant strain according to claim 8, wherein the plasmid obtained in step II is designated pJQ200SK*devR::kan.*

11. The *devR* mutant strain according to claim 8 or 10, wherein step III is effected by electroporation.

12. The *devR* mutant strain according to any of claims 7 to 11 having a size increment of 1.3 kb corresponding to the Km^{R} cassette inserted in the *devR* locus instead of the wild-type *devR* gene having a 3.8 kb signal.

13. The *devR* mutant strain according to any of claims 6 to 12, showing nearly thousand-fold lower bacterial load in animals at 7 weeks post-infection as compared to the parental strain.

## Patentansprüche

1. Verfahren zur Identifizierung eines neuen Ziels zum Gebrauch für die Entwicklung von therapeutischen Modalitäten und Medikamenten, die wirksam sind gegen Tuberkulose enthaltend die folgenden Schritte:
I. Unterbrechung des devR- Gens (Rv3133c), das in einem ~ 3,3 kb *Eco*RI-Hindlll Einschubs eines Plasmids pJT53.34 angeordnet ist,
II. Konstruktion von pJQ200Sk*devR::kan* aus dem unterbrochenen devR-Gen,
III. Einführung des genannten Plasmids in *M. tuberculosis* H37Rv,
IV. auswählen eines einzelnen Crossover-Transformants indikativ für eine Plasmid-Integration auf Middle Brook 7H10 Agar Platten, die Kanamycin enthalten,
V. analysieren derselbigen durch Polymerase-Kettenreationen (polymerase chain reaction - PCR) auf die Anwesenheit von *devR* Km^{R} und Sucrose Resistenz (SacB) Gensequenzen,
VI. die genannten Sequenzen dem Schritt der Southern Analyse zu unterziehen, mit jeder der *dev*R-Probe, *dev*S-Probe und Km^{R}-Gen-Proben, um so *M. tuberkulosis* Dup *devR* zu bestimmen, die Wildarten enthalten und die unterbrochenen Kopien des devR-Locus,
VII. anzüchten von *M. tuberkulosis* Dup *devR* in Middle Brook 7H9 Medium, das Kanamycin und Sucrose enthält,
VIII. ausbringen der genannten gewachsenen *M.tuberculosis* Dup *devR*-Ketten in eine Vielzahl von Platten, die ein 7H10 Medium haben, das Kanamycin und Sucrose darin enthält, um so Kanamycin resistente Transformanten zu erhalten, um so devR-Mutationsketten von *M. tuberculosis* zu erhalten,
IX. die genannten gewachsenen *M. tuberculosis devR* Mutanten dem Schritt einer Southern Hybridisation zu unterziehen gefolgt durch Polymerase-Kettenreaktions-Prozesse für die Bestätigung des Ersatzes von wildartigen *devR* Allelen mit unterbrochenen *devR* Allelen,
X. die genannten *M.tuberculosis devR* Transformanten dem Schritt aussetzen der Polymerase-Kettenreaktions-Analyse auf devR kan unterbrochene Gene,
XI. die genannten devR kan unterbrochenen Gene dem Schritt des Western-Blottings zu unterziehen und immuno elektronischer Mikroskopie zur Bestätigung der funktionellen Unterbrechung der genannten Gene,
XII. Auswertung der Wachstumslebensfähigkeit der Ketten von *M.tubverculosis devR* Mutanten unter Bedingungen von Sauerstoffbegrenzung für *devR-* und *dev*S-Genausdrücke,
XIII. Auswertung der Wachstumslebensfähigkeit der genannten Ketten von *M.tuberculosis devR* Mutanten unter Bedingung von Sauerstoffbegrenzung in aeroben Bedingungen für *devR-* und *dev*S-Genausdrücke,
XIV. die genannten gewachsenen Ketten dem Schritt einer RT-PCR Analyse unterziehen auf Transkriptionen, die erhalten wurden von dem *Rv3134c-devR-devS* Operon,
XV. scannen der genannten Transkriptionen durch Benutzung eines Ultraviolettdurchleuchters und einer Gel-Dokumentation sowie dieselben dem Schritt der densitometrischen Analyse zu unterziehen durch Gebrauch einer Computersoftware,
XVI. testen von *M.tuberculosis devR* Mutationsketten auf Virilität in nichtmenschlichen Tieren.

2. Das Verfahren gemäß Anspruch 1,
wobei das Testen auf Virilität durchgeführt wird an Meerschweinchen und umfasst:
(a) histopathologische Analyse der infizierten Organe des Meerschweinchens infiziert mit *devR* Mutanten und wildartigen Ketten von *M.tuberculosis,* und
(b) rückgewinnen von *M.tuberculosis* aus der Milz von infizierten Tieren und Quantifizierung der Bakterienlast.

3. Das Verfahren gemäß Ansprüchen 1 oder 2,
wobei das genannte *devR* Allele unterbrochen wird durch ein Kanamycinresistenz-(Km^{R})-Gen an einer einzigen *Ppu*Ml Stelle.

4. Das Verfahren gemäß einem der Ansprüche 1 bis 3,
wobei das *devR* Allele herausgetrennt wird als ein Apal-BamHl-Fragment und geklont wird in korrespondierenden Stellen des Plasmids pJQ200SK, um so pJQ200SKdevR::kan zu bilden.

5. Das Verfahren gemäß einem der Ansprüche 1 bis 4,
wobei das Plasmid eingeführt wird in *M.tuberculosis* H37Rv durch Elektroporation.

6. Eine *devR*-Mutationskette von *M*.tuberculosis, bei der das wildartige *devR* Gen unterbrochen wird mit einem antibiotischen *Resistenz-Gen.*

7. Eine *devR*-Mutationskette für *M.tuberculosis,* erhalten durch ein Verfahren gemäß einem der Ansprüche 1 bis 5.

8. Eine *devR* Mutationskette für *M.tuberculosis,* zu erhalten durch ein Verfahren enthaltend die folgenden Schritte:
I. Unterbrechung des wildartigen *devR* Gens (Rv3133c), das an einem a ∼ 3.3 kb *Eco*RI*-Hind*III Einschub eines Plasmid pJT53.34 angeordnet ist, mit einem antibiotischen-Resistenz-Gen an einer *Ppu*MI Stelle,
II. heraustrennen der unterbrochenen *devR* Allele als ein *Apa*I*-Bam*HI Fragment und klonen desselben in die entsprechenden Stellen eines Plasmids pJQ200SK,
III. einführen des Plasmids, das in Schritt II erhalten wurde, in ein wildartiges *M.tuberculosis* H37Rv,
IV. auswählen eines einzelnen Crossover-Transformants indikativ für eine Plasmidintegration,
V. kultivieren des einzelnen Crossover-Transformants, der in Schritt IV gewählt wurde, und auswählen von doppelten Crossover-Transformants, die nicht in der Lage sind, den wildartigen *devR* Typ auszudrücken, wodurch ein *devR* Mutationsstrang von *M.tuberculosis* erhalten wird.

9. Die *devR*-Mutationskette gemäß Anspruch 6 oder 8, bei der das antibiotische Resistenz-Gen ein Kanamycin-Resistenz-Gen ist.

10. Die *devR*-Mutationskette gemäß Anspruch 8, wobei das erhaltene Plasmid in Schritt II bestimmt wird als pJQ200SK*devR::kan.*

11. Die *devR*-Mutationskette gemäß Anspruch 8 oder 10, bei der Schritt 3 bewirkt wird durch Elektroporation.

12. Die *devR*-Mutationskette gemäß einem der Ansprüche 7 bis 11, mit einem Abmessungsinkrement von 1,3 kb entsprechend der Km^{R}-Kassette, die in den *devR* Locus statt des wildartigen *devR* Gens eingeführt wurde, das ein 3.8 kb Signal hat.

13. Die *devR*-Mutationskette gemäß einem der Ansprüche 6 bis 12, dass 7 Wochen nach der Infektion bei Tieren eine fast tausendfach niedrigere Bakterienlast aufweist verglichen mit der Elternkette.

## Revendications

1. Procédé pour l'identification d'une nouvelle cible destinée à être utilisée pour le développement de modalités thérapeutiques et de médicaments efficaces contre la tuberculose, comprenant les étapes suivantes :
I. rupture du gène devR (Rv3133c) localisé dans un insert *Eco*RI-*Hin*dIII de ∼3,3 kb du plasmide pJT53.34,
II. construction de pJQ200Sk*devR::kan* à partir du gène devR rompu,
III. introduction dudit plasmide dans *M tuberculosis* H37Rv,
IV. sélection de transformants à un seul échange, indicateurs d'intégration du plasmide, sur des plaques de gélose Middle Brook 7H10 contenant de la kanamycine,
V. analyse de ceux-ci par la réaction d'amplification en chaîne par polymérase (PCR) pour déterminer la présence de séquences de gènes *devR,* Km^{R} et de résistance au saccharose (SacB),
VI. exposition desdites séquences à l'étape d'analyse de Southem à l'aide de chacune des sondes sonde *devR,* sonde *devS* et sonde du gène Km^{R} de manière à mettre en évidence *M tuberculosis* Dup *devR* contenant le type sauvage et les copies rompues du locus devR,
VII. culture de *M. tuberculosis* Dup *devR* dans du milieu Middle Brook 7H9 contenant de la kanamycine et du saccharose,
VIII. transfert de la dite souche *M tuberculosis* Dup *devR* développée, dans plusieurs plaques comportant un milieu 7H10 contenant de la kanamycine et du saccharose, de manière à obtenir des transformants résistants à la kanamycine, pour l'obtention d'une souche *devR* mutante de *M tuberculosis,*
IX. exposition dudit mutant de *M tuberculosis devR* développé, à l'étape d'hybridation de Southem suivie d'un processus de réaction d'amplification en chaîne par polymérase pour la conformation du remplacement de *devR* allélique de type sauvage par *devR* allélique rompu,
X. exposition desdits transformants *M. tuberculosis devR* à l'étape d'analyse par la réaction d'amplification en chaîne par polymérase pour la détermination du gène devR kan rompu,
XI. exposition dudit gène devR kan rompu à l'étape d'analyse western blot et d'examen par immunomicroscopie électronique pour la confirmation de la destruction de la fonction dudit gène,
XII. évaluation de la viabilité du développement du mutant de la souche *M. tuberculosis devR* dans des conditions de limitation d'oxygène pour l'expression des gènes *devR* et *devS,*
XIII. évaluation de la viabilité du développement dudit mutant de la souche *M tuberculosis devR* dans des conditions de limitation d'oxygène dans des conditions aérobies pour l'expression des gènes *devR* et *devS,*
XIV. exposition de ladite souche développée à l'étape d'analyse par RT-PCR à la recherche de transcrits obtenus à partir de l'opéron *Rv3134c-devR-devS,*
XV. examen desdits transcrits au moyen d'un transilluminateur ultraviolet et d'un système de documentation de gel et exposition de ceux-ci à l'étape d'analyse densitométrique au moyen d'un programme informatique,
XVI. test de la souche *devR* mutante de *M tuberculosis* visant à déterminer la virulence chez des animaux non humains.

2. Procédé selon la revendication 1, dans lequel le test visant à déterminer la virulence est effectué sur des cobayes et comprend :
(a) une analyse histopathologique des organes infectés provenant de cobayes infectés par des souches *devR* mutantes et de type sauvage de *M tuberculosis,* et
(b) la récupération de *M tuberculosis* à partir de rates d'animaux infectés et la détermination quantitative de la charge bactérienne.

3. Procédé selon la revendication 1 ou 2, dans lequel ledit allèle *devR* est rompu par un gène de résistance à la kanamycine (Km^{R}) au niveau d'un unique site *PpuMI.*

4. Procédé selon l'une quelconque des revendications 1 à 3, dans lequel l'allèle *devR* est excisé sous forme d'un fragment *Apa*I*-Bam*HI et cloné dans les sites correspondants du plasmide pJQ200SK de manière à donner pJQ200SK*devR::kan.*

5. Procédé selon l'une quelconque des revendications 1 à 4, dans lequel le plasmide est introduit dans *M. tuberculosis* H37Rv par électroporation.

6. Souche *devR* mutante de *M tuberculosis,* dans laquelle le gène *devR* de type sauvage est rompu par un gène de résistance à un antibiotique.

7. Souche *devR* mutante de *M tuberculosis,* pouvant être obtenue par le procédé tel que défini dans l'une quelconque des revendications 1 à 5.

8. Souche *devR* mutante de *M tuberculosis,* pouvant être obtenue par un procédé comprenant les étapes suivantes :
I. rupture du gène *devR* de type sauvage (*Rv3133c*) localisé dans un insert *Eco*RI*-Hind*III de ∼3,3 kb du plasmide pJT53.34 par un gène de résistance à un antibiotique au niveau d'un unique site *Ppu*MI,
II. excision de l'allèle *devR* rompu, sous forme d'un fragment *Apa*I*-Bam*HI et clonage de celui-ci dans les sites correspondants du plasmide pJQ200SK,
III. introduction du plasmide, obtenu dans l'étape II, dans *M tuberculosis* H37Rv de type sauvage,
IV. sélection de transformants à un seul échange, indicateurs de l'intégration du plasmide,
V. culture de transformants à un seul échange, sélectionnés dans l'étape IV, et sélection de transformants à double échange qui sont incapables d'exprimer le gène *devR* de type sauvage, pour l'obtention d'une souche *devR* mutante de *M tuberculosis.*

9. Souche *devR* mutante selon la revendication 6 ou 8, dans laquelle le gène de résistance à un antibiotique est un gène de résistance à la kanamycine.

10. Souche *devR* mutante selon la revendication 8, dans laquelle le plasmide obtenu dans l'étape II est désigné par *pJQ200SKdevR::kan.*

11. Souche *devR* mutante selon la revendication 8 ou 10, dans laquelle l'étape III est effectuée par électroporation.

12. Souche *devR* mutante selon l'une quelconque des revendications 7 à 11, présentant une augmentation de taille de 1,3 kb correspondant à la cassette Km^{R} insérée dans le locus *devR* au lieu du gène *devR* de type sauvage ayant un signal de 3,8 kb.

13. Souche *devR* mutante selon l'une quelconque des revendications 6 à 12, présentant une charge bactérienne presque mille fois plus faible chez des animaux à 7 semaines après infection, par comparaison avec la souche parentale.
